# EUROPÄISCHE PATENTSCHRIFT

(11) **EP 0 485 563 B1**
(45) Veröffentlichungstag und Bekanntmachung des Hinweises auf die Patenterteilung: **21.12.1994**
(21) Anmeldenummer: 91910296.2
(22) Anmeldetag: 29.05.1991
(51) Int. Cl.: A61K 47/48

(54) **POLYETHERSUBSTITUIERTE TUMORMITTEL**
POLYETHER-SUBSTITUTED TUMOR AGENTS
AGENTS ANTI-TUMORAUX SUBSTITUES AVEC DU POLYETHER

(30) Priorität: 30.05.1990 DE 4017439
(43) Veröffentlichungstag der Anmeldung: 20.05.1992
(73) Patentinhaber: Deutsches Krebsforschungszentrum Stiftung des öffentlichen Rechts, 69120 Heidelberg (DE)
(72) Erfinder: SINN, Hans-J., D-6908 Wiesloch (DE); SCHRENK, Hans-Hermann, D-6721 Zeiskam (DE); MAIER-BORST, Wolfgang, D-6901 Dossenheim (DE); FRIEDRICH, Eckhard, D-6741 Ilbesheim (DE); GRASCHEW, Georgi, D-6900 Heidelberg (DE); WÖHRLE, Dieter, D-2800 Bremen (DE); KLENNER, Thomas, D-6945 Hirschberg (DE)
(74) Vertreter: Müller-Boré & Partner Patentanwälte
(86) Internationale Anmeldenummer: EP9100992
(87) Internationale Veröffentlichungsnummer: WO9118630

(56) Entgegenhaltungen:
- EP-A- 0 205 326
- EP-A- 0 207 557
- EP-A- 0 210 412
- EP-A- 0 304 311
- EP-A- 0 336 879
- WO-A-88/00837
- WO-A-90/15628
- FILE SERVER STN, FILE CHEMICAL ABSTRACTS, Band 112, Nr. 3, 1990, Seite 519, Zusammenfassung Nr. 21146y, , (Columbus, 0hio, US)
- DATABASE JAPS, Band Nr. 115 (C-0696), 5. März 1990 (KYOWA HAKKO KOGYO CO. LTD)
- FILE SERVER STN, FILE CHEMICAL ABSTRACTS, Band 104, 1986, Seite 653, Zusammenfassung Nr. 139757m, (Columbus, 0hio, US), C. PIECHOCKI et al.

## Beschreibung

Die Erfindung betrifft polyethersubstituierte Tumormittel, nämlich die Anwendung von Polyethern als neue synthetische Trägersysteme zur Einschleusung niedermolekularer Verbindungen in Tumoren zur Verbesserung der in vivo-Tumordiagnostik und Tumortherapie mit unterschiedlichen Behandlungsmethoden.

Die heute für diagnostische und therapeutische Zwecke zur Erkennung und Behandlung von Tumoren eingesetzten Substanzen sind in der Regel niedermolekular und werden nur in Ausnahmefällen spezifisch von den neoplastischen Geweben angereichert. Typische Beispiele niedermolekularer Arzneimittel sind.
1. cis-Platin Verbindungen, Tetracycline, Steroide und dergleichen zur Chemotherapie.
2. Porphyrine, Phthalocyanine und Naphthalocyanine zur laserinduzierten Fluoreszenzdiagnostik und photodynamischen Therapie (PDT).
3. Konventionelle Röntgenkontrastmittel wie Gadolinium-DTPA (ein niedermolekularer Metallkomplex) für die Computer-Tomographie (CT) bzw. Magnet-Resonanz Tomographie (MRT).

Um die am gewünschten Ort erforderliche hohe Konzentration eines eingesetzten Mittels zu erzielen, z.B. die im Tumorgewebe erforderlichen Substanzkonzentrationen, müssen daher, soweit dies überhaupt aufgrund der Toxizität der Mittel möglich ist, hohe Anfangsmengen an Verbindung appliziert werden. Die photodynamische Therapie (PDT) und auch die Chemotherapie beruhen im Prinzip auf der Wirkung von weniger als 1 % der verabreichten Gesamtdosis auf das Tumorgewebe, wenn diese Substanzen systemisch appliziert werden. Da somit in der Regel weniger als 1 % der applizierten Dosis den Tumor erreichen, stellen die restlichen 99 % eine zwar nicht gewünschte aber bisher nicht vermeidbare Belastung für die gesunden Organe dar, vor allem von Leber und Nieren.

In der WO-A-90 15 628 wird ein Tetracyclin-Polymer Konjugat beschrieben. Als Polymer wird Polyethylenglykol mit einem Molekulargewicht von 600-20000, vorzugsweise 2000-12000 genannt. Dieses ist endständig mit einem C₁₋₅ Alkyl, vorzugsweise Methyl, substituiert.

Desweiteren beschreibt DATABASE JAPS, Band Nr. 115, JP,A, 1316400 ein CSF, an das 2,4-bis(0-Methoxypolyethylenglykol)-6-chlor-S-triazin über ein oder mehrere Aminogruppen des CSF gebunden ist.

Beide Dokumente beschreiben keinen befriedigenden Weg, Mittel gezielt an einen Ort zu transportieren, wo sie tatsächlich benötigt werden, um dort die erforderliche hohe Konzentration aufzubauen, ohne auch die übrigen Organe sonderlich zu belasten.

Der vorliegenden Erfindung liegt daher die Aufgabe zugrunde, einen Weg aufzuzeigen, mit dem Vorstehendes erreicht werden kann.

Erfindungsgemäß wird dies durch die Derivatisierung aromatischer Hydroxyl- bzw. Aminogruppen von Verbindungen mit Polyethylenglykolen erreicht, deren endständige Hydroxylgruppe mit C₁- C₁₂-Alkygruppen verethert oder verestert ist, wobei eine gewisse Bevorzugung für die C₁-C₈, insbesondere C₁ und C₆-Alkylgruppe sowie die C₁₀-und C₁₂-Alkylgruppe besteht. Die Methoxygruppe ist jedoch am meisten bevorzugt. Für den Fall der Methoxypolyethylenglykole (MPEG) werden Kettenlängen von 5 bis 250 Ethylenoxideinheiten, also n = 5 bis 250 benutzt, vorzugsweise Ketten mit n = 10 bis 200. Dies bedeutet für den Polyethylenglykolrest einschließlich der Methoxygruppe, wobei die Polyethylenglykolkette natürlich auch verzweigt sein kann, ein Molekulargewicht von ca. 250 bis ca. 11000 (n = 5 bis 250), insbesondere ca. 470 bis knapp 9000 (n = 10 bis 200). Da mindestens Zwei solche endständig substituierte Polyethylenglykolketten an die zu derivatisierende Verbindung gebunden werden, ist deren Kettenlänge so zu wählen, daß das gewünschte Gesamtmolekulargewicht entsteht. Methoxypolyethylenglykole vom ungefähren Molekulargewicht 5000 sind besonders bevorzugt. Wenn ein Gesamtmolekulargewicht von über 10000 erwünscht ist oder insbesondere wenn die Polyethylenglykolkette selbst schon ein recht hohes Molekulargewicht hat, das nahe an oder über 10000 liegt, wird in bekannter Weise mit Cyanurchlorid aktiviert, um die Derivatisierung durchzuführen. Derartig mit Cyanurchlorid aktivierte Methoxypolyethylenglykole werden hier CMPEG genannt.

In der zu derivatisierenden Verbindung, üblicherweise ein Cytostatikum, (tumoraktive Substanz) oder eine für die Diagnostik geeignete Verbindung (Tumordiagnostik-Substanz), können neben phenolischen Hydroxyl- bzw. Aminogruppen, wobei mindestens zwei vorliegen sollen, auch aliphatische Aminogruppen solcher niedermolekularer pharmazeutischer Verbindungen für diese Derivatisierung benutzt werden, wobei sich die Aminogruppen jedoch nicht bei O-Jodverbindungen eignen, da Jod sonst verschoben oder abgespalten wird.

Ein erfindungsgemäßes Derivat umfaßt also eine tumoraktive oder Tumordiagnostik-Substanz, die sich dadurch kennzeichnet, daß sie
- mindestens zwei phenolische Hydroxyl- und/oder mindestens zwei phenolische Aminogruppen,
   oder
- mindestens eine phenolische Hydroxyl- und/oder mindestens eine phenolische Aminogruppe und mindestens eine aliphatische Aminogruppe aufweist, und diese Gruppen mit Polyethylenglykolketten substituiert sind, deren Polymerisationsgrad n 5 bis 250 ist und deren endständige Hydroxylgruppe durch C₁ - C₁₂-Alkylester oder/ -ether substituiert ist, wobei die Substanz jeweils durch mindestens zwei solcher Polyethylenglykolketten substituiert ist.

Gesamtmolekulargewichte der mit Polyethylenglykolresten derivatisierten Cytostatika von 10000 und mehr sind deutlich bevorzugt, da Verbindungen dieser Größenordnung nicht so leicht durch die Niere ausgeschieden werden (die Ausschlußgrenze der Niere liegt beim Molekulargewicht ca. 20000).

Wenn also z.B. 4 Polyethylenglykolreste gebunden werden und das Molekulargewicht etwas über 10000 betragen soll, dann soll das Molekulargewicht jeder Polyethylenoxidkette (die Methoxy- oder Alkoxy- oder Estergruppe wird hier vernachlässigt), mindestens ca. 2000 bis 2500 betragen, um ein Gesamtmolekulargewicht von gut 10000 für die derivatisierte Verbindung zu ergeben.

Durch die Kettenlänge der Polyethylenglykolkette oder durch deren Verzweigung wird mit Serumalbumin ein Gesamtblock gebildet, der gut im Körper bleibt und nicht leicht wie üblich ausgeschieden wird, sondern sich auch gut auf den zu behandelnden oder zu diagnostizierenden Tumor konzentriert.

Durch diese Blockbildung mit Serumalbumin ist es nicht erforderlich, daß das Gesamtmolekulargewicht der zu derivatisierenden Verbindungen einschließlich der Polyethylenglykolketten über 20000 beträgt, obwohl man auch natürlich solche hohen Molekulargewichte wählen kann, wenn man sich nicht auf diese Blockbildungmit Serumalbumin verlassen will. So ergibt z.B. der Einbau von vier der bevorzugten Methoxypolyalkylenglykolketten vom Molekulargewicht von ca. 5000 in eine zu derivatisierende Verbindung ein Gesamtmolekulargewicht von gut 20000.

Es ist bekannt, Polyethylenglykol an die verschiedensten Substanzen anzuknüpfen, um gewisse Wirkungen zu erreichen. So zeigen Abuchowski und Mitarbeiter in J.Biol.Chem. 252, 3578 (1977) die Veränderung immunologischer Eigenschaften von Rinderserumalbumin durch die kovalente Anknüpfung von Polyethylenglykol, Beauchamp und Mitarbeiter zeigen in Analytical Biochemistry 131, 25 (1983) eine Synthese von Polyethylenglykol-Proteinadukten sowie Effekte auf die Funktion, die Rezeptorerkennung und die Clearance von Superoxide Dismutase, Lactoferrin und alpha-2-Macroglobulin; Inada und Mitarbeiter zeigen in TIBTECH, Juli 1966, 190, die Anwendung von Polyethylenglykol-modifizierten Enzymen in biotechnologischen Prozessen, nämlich in organischen Lösungsmitteln löslichen Enzymen; Takahashi und Mitarbeiter zeigen in Biochem. und BioPhys. Res. Communications, 138, 283 (1986) Polyethylenglykol-modifiziertes Hämin mit Peroxidaseaktivität in organischen Lösungsmitteln; Jackson und Mitarbeiter zeigen in Analytrical Biochemistry 165, 114 (1987 die Synthese, Isolierung und Characterisierung von Konjugaten von Ovalbumin mit Monomethoxypolyethylenglykol unter Verwendung von Cyanurchlorid als Kupplungsmittel und Kay und Lilly zeigen in Biochemica et Biophysica Acta 198, 276 (1970) die chemische Bindung von Chymotrypsin zu wasserunlöslichen Polymeren unter Verwendung von 2-Amino-4,6-Dichlor-s-Triazin.

Alle diese Arbeiten befassen sich jedoch nicht mit dem hier anstehenden Problem, das insbesondere für die Tumortherapie von Bedeutung ist.

Durch die Derivatisierung der Verbindung mit MPEG bzw. CMPEG wird:
1. Generell eine Erhöhung der Hydrophilie erzeugt, wobei im physiologischen pH-Bereich eine hohe Wasserlöslichkeit erzielt wird.
2. Eine starke Bindung an Plasmaproteinen erzeugt, was insbesondere die Nierenausscheidung stark verzögert.
3. Eine Unterdrückung der Erkennungs- und Abfangmechanismen in der Leber gegenüber Fremdsubstanzen erreicht und
4. eine wesentliche Steigerung der Stabilität jodhaltiger Verbindungen erzielt.

Dadurch sind folgende Vorteile möglich:
1. Die Erzeugung hoher Hydrophilie (Wasserlöslichkeit) aller Substanzen beinhaltet, daß alle Verbindungen in isotonen wässrigen Medien ohne weitere Lösungsvermittler anwendbar sind.
2. Eine starke Bindung der MPEG- bzw. CMPEG-Derivate an Plasmaproteine verhindert eine schnelle Ausscheidung der Fremdsubstanzen über die Nieren.
3. Die Unterdrückung der Erkennungs- und Abfangmechanismen in der Leber gewährleistet in Kombination mit der unterdrückten Nierenausscheidung eine lange Verweilzeit im Blut, was gleichzeitig wesentlich erhöhte Anreicherungen im Tumor zur Folge hat.
4. Die Stabilisierung von (radio-)jodhaltigen Verbindungen verhindert die Abspaltung von (radio-)Jod im Organismus was einerseits für die Reduktion der Strahlenbelastung der Schilddrüse mit Radiojod von großer Bedeutung ist, andererseits bei Patienten mit einer Jodallergie erheblich verminderte Risiken beinhaltet.
5. Die wesentliche Steigerung der Tumoranreicherungsraten (bei Tierversuchen in der Regel 30%, ja bis zu 50 % der gesamt applizierten Dosis und mehr) erlauben eine generelle Reduzierung der für diagnostische oder therapeutische Maßnahmen notwendigen Substanzmengen, was wiederum eine Verminderung der Ganzkörperbelastung zur Folge hat. Bei dem schnell wachsenden Melanom wurde schon eine Konzentration von 80 % der Wirksubstanz an der zu behandelnden Stelle beobachtet.

Die Anreicherung, die sich insbesondere bei den radioaktiv markierten Substanzen deutlich in den Aufnahmen zeigt, ist außerordentlich überraschend. Es besteht nun die Möglichkeit, Substanzen insbesondere für die Diagnostik und die Therapie von Tumoren, gezielt an Ort und Stelle zu bringen.

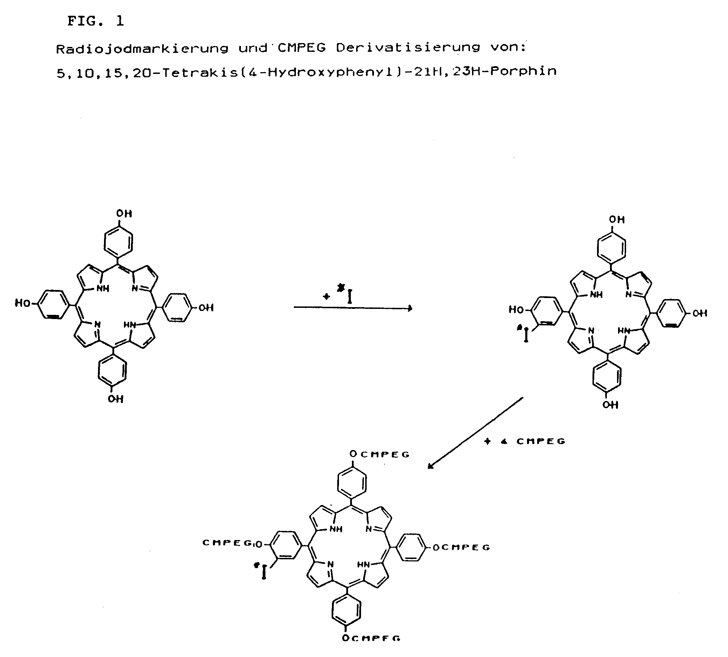

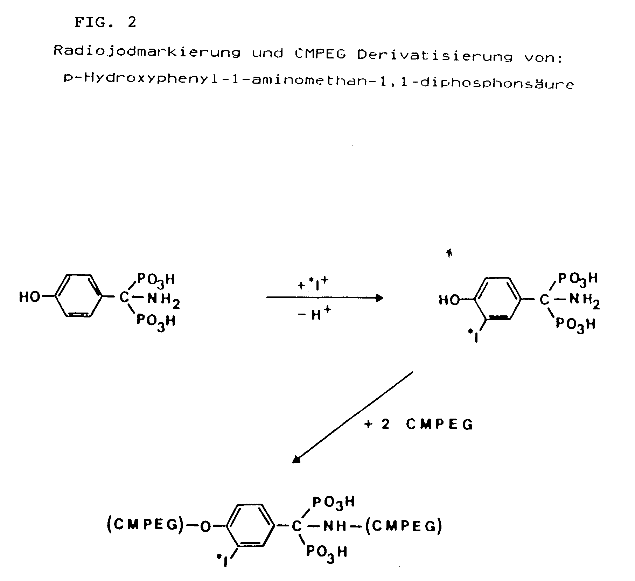

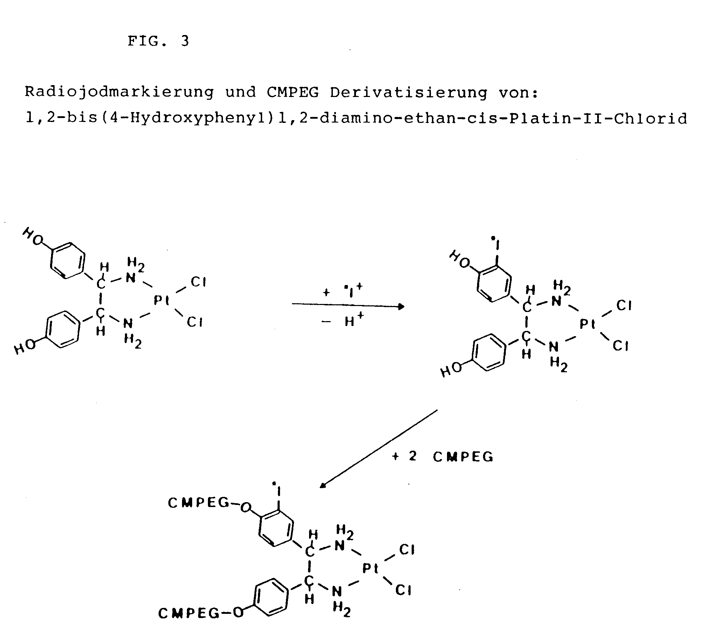

Beispiele für polyetherderivatisierte Substanzen, die alle eine hohe spezifische Anreicherung im Tumorgewebe aufweisen und eine wesentlich reduzierte Belastung von Normalgeweben bzw. Organen zur Folge haben, sowie deren Einsatzmöglichkeiten sind die folgenden:
1. Porphine, Phthalocyanine und Naphthalocyanine, die vom Typ her bekannt sind, für die laserinduzierte Fluoreszenzdiagnostik und photodynamische Therapie (PDT) von Tumoren. Als radiojodmarkierte Verbindung zur nuklearmedizinischen Lokalisation unbekannter Metastasen, zur Bestimmung des optimalen Zeitpunkts für eine PDT sowie für eine mögliche Radiojodtherapie. Reaktionsschemata für die Radiojodmarkierung sowie CMPEG-Derivatisierung sind in Fig. 1 für 5,10,15,20-Tetrakis(4-Hydroxyphenyl)-21H,23H-Porphin gezeigt.
2. Radiojodmarkierte Diphosphonate zur nuklearmedizinischen Lokalisation von Tumoren und/oder Metastasen bzw. deren Radiojodtherapie. Reaktionsschemas für die Radiojodmarkierung sowie CMPEG-Derivatisierung sind in Fig. 2 für p-Hydroxyphenyl-1-aminomethan-1,1-diphosphonsäure gezeigt.
3. Cis-Platin und cis-Platin-abkömmlinge, Tetracycline und Steroide sowie ähnliche Verbindungen für die Chemotherapie. Radiojodmarkierte Produkte dienen zur nuklearmedizinischen Erfassung der individuellen Substanzverteilungsmuster, Reaktionsschemata für die Radiojodmarkierung sowie CMPEG-Derivatisierung sind in Fig. 3 für 1,2-bis/4-Hydroxyphenyl)1,2-diamino-ethan-cis-Platin-II-Chl orid gezeigt.
4. Mit Radiojod, Radiobrom oder mit radioaktiven Metallionen markierte Peptide, Porphine, Phthalocyanine, Naphthalocyanine und ähnliche Verbindungen zur positiven Darstellung von Tumoren mit planar bzw. tomographisch abbildenden gamma-Kamera
5. Hoch jodierte Verbindungen wie z.B.: Porphine, oligomere Tyrosine etc. zur positiven Darstellung von Tumoren mit Hilfe der Computer-Tomographie (CT).
6. Metallkomplexe wie z.B.: Gadolinium-Phthalocyanine zur positiven Tumordarstellung mit Hilfe der Magnet-Resonanz-Tomographie (MRT).
7. Radiosensibilisatoren wie z.B.C. Nitroimidazol als unterstützende Maßnahme bei der Strahlentherapie von Tumoren mit externen oder tumorinkorporierten Strahlenquellen z.B. bei der PDT, die auch schon unter Punkt 1 erörtert ist.
8. Luminole zur intratumoralen Erzeugung von Chemilumineszenz und ihre Anwendung zur direkten Phototherapie neoplastischer Gewebe.
9. Borsäureester für die Neutroneneinfangtherapie von Tumoren.
10. Dysprosium 164 für Phthalocyanine (nicht z.B. für Porphyrine, da Dy dafür zu groß ist). Dy¹⁶⁴, das letzte stabile Isotop, fängt thermische Neutronen ein und emittiert β, wobei es in Dy¹⁶⁴ übergeht.

Die Polyethylenglykolreste sind derart, daß die Gesamtverbindungen ein Molekulargewicht von 2000 bis 10000 oder darüber haben. Nachdem mindestens zwei solcher Reste an das Cytostatikum gebunden werden sollen, bedeutet dies ein Mindestmolekulargewicht für die z.B. Methoxypolyethylenglykole von knapp 1000. Besonders bevorzugt istein Molekulargewicht von etwa 5000. Bei diesen Polyethylenoxidresten ist ein endständiges Hydroxyl durch eine C₁ - C₁₂-Alkylether- oder -estergruppe substituiert. Die Methoxyverbindung ist dabei bevorzugt. Methoxypolyethylenoxide mit einem Molekulargewicht von etwa 5000 sind im Handel erhältlich und können, evtl. nach üblicher bekannter Reinigung, direkt für die Synthese der erfindungsgemäßen Derivate von Cytostatika verwendet werden. Wenn ein solches Mittel z.B. mit Jod markiert werden soll, dann wird zuerst das Jodatom eingeführt und dann wird, wie üblich, mit einem Linker, vorzugsweise Cyanurchlorid, die Polyoxyethylenkette angefügt.

Das Gesamtmolekulargewicht der erfindungsgemäßen Derivate von Cytostatika beträgt vorzugsweise mehr als 10 000. Wenn man z.B. ein Porphin gemäß Fig. 1 verwendet, dessen Molekulargewicht ca. 678 beträgt, so werden hier 4 Methoxypolyethylenoxidreste mit einem Molekulargewicht von etwa 5000, jedoch mindestens etwa 2500 angeheftet.

Bei der Verbindung gemäß Fig. 2, der gezeigten Diphosphonsäureverbindung, sollten daher zwei Methoxypolyethylenoxidreste vom Molekulargewicht etwa 5000 oder noch besser 6000 angeheftet werden.

Wie erwähnt, gewährt diese Polyethylenglykolsubstitution durch die Kettenlänge und/oder die Verzweigung der Kette die Bildung eines Gesamtblockes mit Serumalbumin, dessen Größe die Ausschlußgrenze der Niere übersteigt.

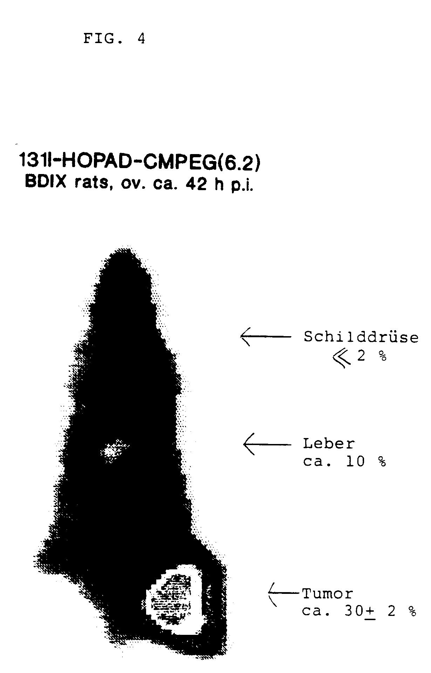

Die Konzentration des verabreichten Mittels, wenn dieses ein Phthalocyanin ist, ist im Tumor so hoch, daß man den Tumor blaugefärbt sieht. Interessant ist, daß der Farbstoff nur in vitalem Tumorgewebe abgelagert wird, nicht jedoch im abgestorbenen Gewebe, wie dies die beigefügte Fig. 4 zeigt.

Die folgenden Beispiele erläutern die Erfindung und zeigen die Herstellung der derivatisierten Cytostatika:

### Beispiel 1

### Herstellung von radiojodmarkiertem und CMPEG-derivatisiertem TOP

Zu 100 µg Tetra-(4-Hydroxyphenyl)-Porphin (TOP), gelöst in 100 µg Dimethylacetamid, gibt man 5-20 µl einer alkalischen Lösung (pH 8-11) von Radiojod (Jodid) mit einer Aktivität von 18,5 MBq (500 µCi) bis 185 MBq (5 mCi) und 3-10 µg N-Chlorsuccinimid (NClS), gelöst in 2-5 µl Acetonitril. Nach einer Reaktionszeit von 20-30 min fügt man 4 mg alpha-Dichlorotriazin-Ω-methoxy-polyethylenglykol (n = 100 -110, also 100 -110 Ethylenglykoleinheiten) gelöst in 40 µl Dioxan und 100 µl 0,17 M Natriumbicarbonatlösung hinzu. Nach einer Reaktionszeit von 20-30 min wird das Reaktionsgemisch mit dest. Wasser auf 200 µl aufgefüllt und die organischen Lösungsmittel über eine Ultrafiltrationseinheit mit einer Ausschlußgrenze von 5 kD abgetrennt. Es ist zu beachten, daß als Lösungsmittel ein organisches Lösungsmittel mit hoher DK verwendet wird.

### Analytik:

### Dünnschichtchromatographie:

Platten: Kieselgel 60 (5 x 20 cm)
(ohne Fluoreszenzindikator)
Laufmittel: Methanol, 10 % NH₄acetat in Wasser (1/1)
Laufstrecke: 10 cm
Rf-Werte:

| | |
|---|---|
| TOP | 0,95-0,98 |
| TOP-CMPEG | 0,0 |

### Beispiel 2

### ¹³¹I-Markierung und CMPEG-Derivatisierung von: p-Hydroxyphenyl-1-aminomethan-1,1-diphosphonsäure (HOPAD)

zu 100 µl einer Lösung von HOPAD in 0,17 M NaHCO₃ (1 mg/ml) fügt man 2-5 mCi (74-185 MBq) einer ¹³¹I-Na-Jodid-Lösung (10-25 µl) und 3-7,5 µg N-Bromosuccinimid (NBS), glöst in 0,17 M NaHCO₃ (1 mg/ml) hinzu. Nach einer Reaktionszeit von 10 min bei Raumtemperatur beträgt die durchschnittliche ¹³¹I-Einbaurate 98 %. Man fügt nun in 1,4-Dioxan gelöstes CMPEG (n = 100-110) (100 mg/ml) in einem molaren Verhältnis von 3:1 (etwa 5,5 mg) hinzu. Nach einer Reaktionszeit von etwa 30 min wird nicht gebundenes ¹³¹I und nicht derivatisiertes HOPAD durch Ultrafiltration oder Dialyse abgetrennt.

### Beispiel 3

### Darstellung von Tetra-(4 Amino-phenoxy)-phthalocyanin -CMPEG (TAPPC-CMPEG)

Eine Lösung von TAPPC (1 mg/ml) in Tetrahydrofuran (THF) wird bei Zimmertemperatur mit einem fünffachen molaren Überschuß a n CMPEG 5000 (100 mg CMPEG pro ml Dioxan) und NaHCO₃ (O,13 M in Wasser) versetzt. Nach der Entfernung des größten Teils der organischen Lösungsmittel im Vakuum wird die Reaktionsmischung mit destilliertem Wasser verdünnt und das Reaktionsprodukt über eine Ultrafiltration (Ausschlußgrenze 10 000 D) von restlichen organischen Lösungsmitteln, Salzen und Wasser getrennt. Nach einem zweiten Waschvorgang und einer anschließenden Sterilfiltration ist der neue Fotosensibilisator applikationsfertig.

Es ist wesentlich, daß dieser Fotosensibilisator eine sehr hohe fotodynamische Aktivität bis über 720 nm zeigt, wobei kaum "bleaching" festzustellen ist.

### Beispiel 4

### Anreicherungsfähigkeit erfindungsgemäßer Verbindungen in Tumoren

Es wurden folgende Verbindungen untersucht:

### Abkürzungen:

TOP = Tetra-(hydroxyphenyl)-prophin
TOPPC = Tetra-(hydroxy-phenoxy)-phthalocyanin
TAPPC = Tetra-(4 Amino-phenoxy)-phthalocyanin-CMPEG
HOPAD = Hydroxyphenyl-aminomethan-diphosphonat
HPBT = Hematoporphyrin-bis-tyrimid
CMPEG = alpha-dichloro-triazin-Ω-methoxy-polyethylenglykol (n = 100 - 110)
AMPEG = alpha-amino-Ω-methoxy-polyethylenglykol (n = 100-110)
In der gleichen Weise wie in Beispiel 1 für ¹³¹I-Tetra(hydroxyphenyl)-porphin-(CMPEG)₄ (1)a) gezeigt, wurden hergestellt:
1)b ¹¹¹-In-Tetra-(hydroxyphenyl)-porphin - (CMPEG)₄
1)c ¹¹¹-In-Tetra-(hydroxyphenoxy)-phthalocyanin-(CMPEG)₄,
1)d ¹³¹-I-Tetra-(hydroxyphenoxy)-phthalocyanin- (DMPEG)₄
1)e ¹³¹-I-Hematoporphyrin-bis-tyrimid-(CMPEG)₂ und
1)f ¹³¹-I-Hydroxyphenylaminomethandiphosphonat- (CMPEG)₂ ,
die alle Anwendung in der Nuklearmedizin finden.

Für die Anwendung in der Fluoreszenzdiagnostik und der photodynamischen Therapie wurden in entsprechender Weise hergestellt.
2a) TOP-(CMPEG)₄
2b) TOPPC-(CMPEG)₄
2d) HPBT-(CMPEG)₂
Die folgenden Verbindungen für NMR-Untersuchungen wurden ebenfalls in entsprechender Weise hergestellt:
3a) Mn-TOP-(CMPEG)₄
3b) Gd-TOPPC-(CMPEG)₄.

Schließlich wurden für CT-Untersuchungen die folgenden Verbindungen gemäß Beispiel 2 hergestellt:
4a) Tri-jod-phenol-CMPEG
4b) Tri-jod-benzoesäure-AMPEG
4c) Iodoxaminsäure-(AMPEG)₂
Die Anreicherung (Konzentrierung) dieser Mittel wurde an folgenden experimentellen Tumoren und an folgenden Tiermodellen geprüft:

| Tumoren | Tiermodell |
|---|---|
| a) experimentelle Tumoren | |
| Ovarialcarcinom (O-342) | Ratte, BD IX |
| Walker Carcinosarcom | Ratte, Wistar |
| Yoshida Hepatom | Ratte,Wistar |
| Novikoff Hepatom | Ratte, Wistar |
| Osteosarcom | Ratte, SD curly |
| b) Xenotransplantate, human | Nacktmaus |
| CX 1 : Coloncarcinom | " |
| LX 1 : axillare Lymphknotenmetastase von einem Lungencarcinom | " |
| MX 1 : Mamacarcinom | " |
| HS 1 : Osteosarcom | " |
| MR I-H-186 : Carcinom corpus uteri | " |
| HeLa : Cervixcarcinom | " |
| EI 28 : Blasencarcinom | " |
| GXF-96 : Magencarcinom | " |

Alle diese experimentellen Tumoren und Xenotransplantate wurden mit den Verbindungen a und c gemäß Beispiel 1 (also 1a und 1c) und drei davon, nämlich das Walker Carcinosarcom, das Osteosarcom und das Ovarialcarcinom (O-342) auch mit dem Diphosphonat gemäß Beispiel 2 untersucht.

Bei allen diesen Anwendungsversuchen wurden immer 300 µC = 11,1 MBq verabreicht. ES zeigten sich mit den eingesetzten Mitteln, nämlich 1a und 1c und die Verbindung aus Beispiel 2 eine Konzentration von mindestens 30 % des Mittels an der gewünschten Stelle, also am Tumor. Die beigefügte Fig. 4 zeigt das Bild einer BDIX-Rattemit Ovarialcarcinom 42 h nach Injektion, die mit ¹³¹J-HOPAD-CMPEG, also ¹³¹J-Hydroxyphenyl-1-aminomethan-1,1-diphosphonat- Cyanurchlorid aktiviertes Polyethylenglykol (n = ca. 100 bis 110) in einer Menge von 300 µC behandelt war. Das Bild zeigt, daß ganz unten, im Tumor ca. 30 +/- 2 % des Mittels konzentriert sind, während in der Mitte die Leber ca. 10 % zeigt und die Schilddrüse, die oberhalb der Leber sichtbar ist, weit weniger als 2 % des Mittels aufgenommen hat. Diese %-Angaben beziehen sich auf die Gesamtmenge an die mit radiojadmarkierten Mittel im Gesamtkörper der Ratte.

Normal ist bei Verarbreichung üblicher Cytostatika, also von Cytostatika, die nicht mit Polyethylenglykolderivat derivatisiert sind, eine Konzentration im Tumor, die etwa so hoch ist, wie sie hier in der Schilddrüse auftritt, d.h. 0,05 bis 0,5 % der gesamten applizierten Menge, wobei jedoch bis zu 90 % der verabreichten Menge in der Leber vorliegen, was natürlich zu einer enormen Belastung dieses Organs führt, eine Belastung, die nicht unerwünscht, sondern auch ausgesprochen schädlich ist.

Die Derivatisierung üblicher Cytostatika, die
- mindestens zwei phenolische Hydroxyl- und/oder mindestens zwei phenolische Aminogruppen,
   oder
- mindestens eine phenolische Hydroxyl- und/oder mindestens eine phenolische Aminogruppe und mindestens eine aliphatische Aminogruppe aufweisen,
mit höher molekularen
Polyoxyethylenglykolen mit blockierter Endgruppe führt somit zu einer wesentlichen Konzentrierung des Mittels im vitalen Tumorgewebe und somit zu einer wesentlich besseren Wirkung am gewünschten Ort und einer wesentlichen Entlastung von Leber und Schilddrüse, was natürlich die Nebenwirkungen solcher Cytostatika bedeutend herarbsetzt.

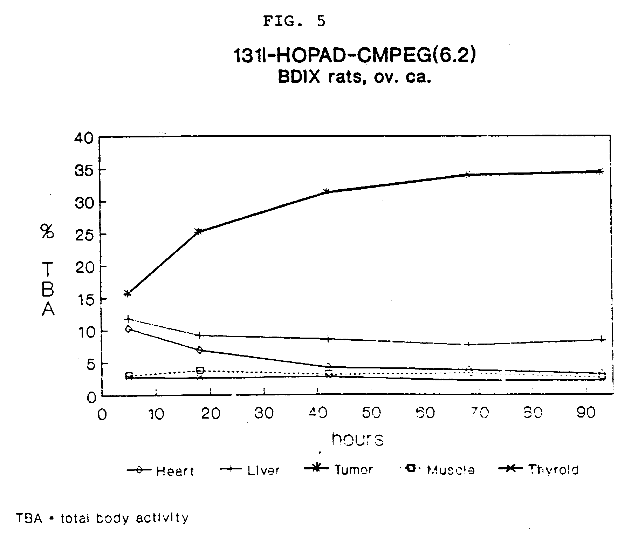

Die beigefügte Fig. 5 zeigt die Messung einer mit ¹³¹I TOP-(CMPEG)4 behandelten BD IX Ratte (auch hier Ovarialcarcinom). Es zeigt sich ein ähnliches Ergebnis wie das von Fig. 4.

Von der gesamten Körperaktivität an radioaktivem Jod finden sich nach 150 h etwa 23 % im Tumor, etwa 12 % in der Leber, etwa 5 % im Blut und etwa 3 - 4 % jeweils in den Muskeln und in der Schilddrüse. Der Rest ist natürlich, wie auch bei der Aufnahme von Fig 4, über den restlichen Körper verteilt.

Auch hier ist eine starke Konzentration im Tumor und eine wesentliche Entlastung der Leber und der Schilddrüse festzustellen, da auch bei dieser nicht derivatisierten Verbindung gilt, da auch bei deren Verabreichung höchstens soviel im Tumor enthalten ist, wie in der Schilddruse.

## Patentansprüche

1. Tumoraktive oder Tumordiagnostik-Stubstanz mit bevorzugter Anreicherung im Tumor, dadurch gekennzeichnet, daß sie
- mindestens zwei phenolische Hydroxyl- und/oder mindestens zwei phenolische Aminogruppen,
oder
- mindestens eine phenolische Hydroxyl- und/oder mindestens eine phenolische Aminogruppe und mindestens eine aliphatische Aminogruppe aufweist,
und diese Gruppen mit Polyethylenglykolketten substituiert sind, deren Polymerisationsgrad n 5 bis 250 ist und deren endständige Hydroxylgruppe durch C₁ - C₁₂-Alkylester oder/ -ether substituiert ist, wobei die Substanz jeweils durch mindestens zwei solcher Polyethylenglykolketten substituiert ist.

2. Tumoraktive oder Tumordiagnostik-Substanz nach Anspruch 1, dadurch gekennzeichnet, daß die Ω-substituierten Polyethylenglykolketten eine Kettenlänge von n = 10 - 200, insbesondere von 100 bis 110 haben.

3. Tumoraktive oder Tumordiagnostik-Substanz nach Anspruch 1 oder 2, dadurch gekennzeichnet, daß die mit Polyethylenglykolketten substituierte Grundsubstanz eine cis-Platinverbindung, ein Steroid, ein Porphin, ein Phthalocyanin, ein Naphthalocyanin oder ein konventionelles Röntgenkontrastmittel ist.

4. Verfahren zur Herstellung der tumoraktiven oder Tumordiagnostik-Substanz nach Anspruch 1 bis 3, dadurch gekennzeichnet, daß man die Grundsubstanz in an sich bekannter Weise mit einem Polyethylenglykol, dessen endständige Hydroxylgruppe mit einer C₁ - C₁₂-Alkylester- oder -ethergruppe substituiert ist, in einem geeigneten Lösungsmittel umsetzt und in an sich bekannter Weise die so derivatisierte Substanz isoliert.

5. Verfahren nach Anspruch 4, dadurch gekennzeichnet, daß man bei Verwendung von Polyethylenglykolketten mit einem Molekulargewicht von über 5000, insbesondere über 10000, die Reaktion mit der tumoraktiven oder Tumordiagnostik-Substanz mittels Cyanurchlorid in an sich bekannter Weise aktiviert.

6. Verwendung von C₁-C₁₂-Omega-Alkylester oder -ether-Polyethylenglykol (Polymerisationsgrad 5 - 250) substituierter niedermolekularer tumoraktiver oder Tumordiagnostik-Substanz mit
- mindestens zwei Phenolischen Hydroxyl- und/oder mindestens zwei phenolischen Aminogruppen,
- oder
- mindestens einer phenolischen Hydroxyl- und/oder mindestens einer phenolischen Aminogruppe und mindestens einer aliphatischen Aminogruppe zur Herstellung eines Mittels mit bevorzugter Anreicherung im/am Tumorgewebe für den Einsatz in der Nuklearmedizin, der Chemotherapie, der Fluoreszenzdiagnostik und der photodynamischen Therapie, sowie in NMR- und CT-Untersuchungen, wobei die Substitution an der phenolischen Hydroxyl- und/oder phenolischen Aminogruppe und/oder der aliphatischen Aminogruppe mit Ausnahme bei O-Jod erfolgt ist.

7. Verwendung von Polyethylenglykol-substituierten Porphinen, Phthalocyaninen und Naphthalocyaninen, die gegebenenfalls radiomarkiert sind, nach Anspruch 6.

8. Verwendung von Polyethylenglykol-substituierten radiojodmarkierten Diphosphonaten nach Anspruch 6, insbesondere zur Radiojodtherapie mit J¹³¹ und J¹²⁵, vorzugsweise zuerst mit J¹³¹ und dann mit J¹²⁵.

9. Verwendung von Polyethylenglykol-substituierten cis-Platinabkömmlingen und Steroiden nach Anspruch 6.

10. Verwendung von Polyethylenglykol-substituierten und radiojodmarkierten Diphosphonaten nach Anspruch 6.

11. Verwendung von Polyethylenglykol-substituierten cis-Platinabkömmlingen und Steroiden nach Anspruch 6.

12. Verwendung von Polyethylenglykol-substituierten und radiojod-, radiobrom- oder mit radioaktiven Metallionen aktivierten Peptiden, Porphinen, Phthalocyaninen und Naphthalocyaninen nach Anspruch 6.

13. Verwendung von Polyethylenglykol-substituierten hochjodierten Verbindungen, insb. Porphinen und polygomeren Tyrosinen nach Anspruch 6.

14. Verwendung von Polyethylenglykol-substituierten Metallkomplexen, insb. Gadolinium, Phthalocyaninen und Naphthalocyaninen,nach Anspruch 6.

15. Verwendung von Polyethylenglykol-substituierten Metallkomplexen, insb. Gadolinium-Phthalocyaninen, nach Anspruch 6.

16. Verwendung von Polyethylenglykol- substituierten Radiosensibilisatoren, insb. Nitroimidazol, nach Anspruch 6.

17. Verwendung von Polyethylenglykol-substituierten Luminolen nach Anspruch 6.

18. Verwendung von Polyethylenglykol-substituierten Borsäureestern nach Anspruch 6.

19. Verwendung von Dy¹⁶⁴-markierten Verbindungen, insbesondere Phthalocyaninen und Naphthalocyaninen,nach Anspruch 6.

## Claims

1. A tumor-active or tumor-diagnostic substance having preferred accumulation in the tumor, characterized in that it has
- at least two phenolic hydroxyl and/or at least two phenolic amino groups,
or
- at least one phenolic hydroxyl and/or at least one phenolic amino group and at least one aliphatic amino group
and these groups are substituted with polyethylene glycol chains whose polymerization degree n is 5 to 250 and whose temrinal hydroxyl group is substituted by C₁-C₁₂ alkyl ester or ether, the substance being substantited by two such polyethylene glycol chains each.

2. Tumor-active or tumor-diagnostic substance according to claim 1, characterized in that the Ω- substituted polyethylene glycol chains have a chain length of n = 10 - 200, particularly 100 to 110.

3. Tumor-active or tumor-diagnostic substance according to claim 1 or 2, characterized in that the basic substance substituted with polyethylene glycol chains is a cis-platin compound, a steroid, a porphin, a phthalocyanine, a naphthalocyanine or a conventional X-ray contrast agent.

4. A process for the production of the tumor-active or tumor-diagnostic substance according to claims 1 to 3, characterized in that the basic substance is reacted in a manner known per se with a polyethylene glycol whose terminal hydroxyl group is substituted with a C₁-C₁₂ alkyl ester or ether group in a suitable solvent and that the thus derivatized substance is isolated in a manner known per se.

5. The process according to claim 4, characterized in that, using polyethylene glycol chains having a molecular weight of over 5,000, particularly over 10,000, the reaction with the tumor-active or tumor-diagnostic substance is activated by means of cyanuric chloride in a manner known per se.

6. Use of low-molecular tumor-active or tumor-diagnostic substance substituted with C₁-C₁₂ omega-alkyl ester or ether polyethylene glycol (polymerization degree 5 - 250) and comprising
- at least two phenolic hydroxyl and/or at least two phenolic amino groups,
- or
- at least one phenolic hydroxyl and/or at least one phenolic amino group and at least one aliphatic amino group for the production of a preparation having preferred accumultation in/at the tumor tissue for use in nuclear medicine, chemotherapy, fluorescence diagnosis and photodynamic therapy as well as in NMR and CT examinations, wherein the substitution has taken place at the phenolic hydroxyl and/or phenolic amino group and/or the aliphatic amino group, an exception being in the case of O-iodine.

7. Use of polyethylene glycol-substituted porphins, phthalocyanines and naphthalocyanines, which are optionally radioactively labeled, according to claim 6.

8. Use of polyethylene glycol-substituted radioiodine-labeled diphosphonates according to claim 6, particularly for radioiodine therapy using I¹³¹ and I¹²⁵, preferably using initially I¹³¹ and then I¹²⁵.

9. Use of polyethylene glycol-substituted cis-platin derivatives and steroids according to claim 6.

10. Use of polyethylene glycol-substituted and radioiodine-labeled diphosphonates according to claim 6.

11. Use of polyethylene glycol-substituted cis-platin derivatives and steroids according to claim 6.

12. Use of polyethylene glycol-substituted and with radioiodine, radiobromine or radioactive metal ions activated peptides, porphins, phthalocyanines and naphthalocyanines, according to claim 6.

13. Use of polyethylene glycol-substituted, highly iodinated compounds, particularly porphins and polygomeric tyrosines, according to claim 6.

14. Use of polyethylene glycol-substituted metal complexes, particularly gadolinium, phthalocyanines and naphthalocyanines according to claim 6.

15. Use of polyethylene glycol-substituted metal complexes, particularly gadolinium-phthalocyanines, according to claim 6.

16. Use of polyethylene glycol-substituted radiosensitizers, particularly nitroimidazole, according to claim 6.

17. Use of polyethylene glycol-substituted luminols according to claim 6.

18. Use of polyethylene glycol-substituted boric-acid esters according to claim 6.

19. Use of Dy¹⁶⁴-labeled compounds, particularly phthalocyanines and naphthalocyanines, according to claim 6.

## Revendications

1. Substance réagissant vis-à-vis d'une tumeur ou substance pour diagnostiquer une tumeur, de préférence concentrée dans ladite tumeur, caractérisée en ce qu'elle contient :
- au moins deux groupes hydroxyles phénoliques et/ou au moins deux groupes amino phénoliques, ou
- au moins un groupe hydroxyle phénolique et/ou au moins un groupe amino phénolique et au moins un groupe amino aliphatique,
et que ces groupes sont substitués par des chaînes de polyéthylèneglycol dont le degré de polymérisation n vaut de 5 à 250 et dont le groupe hydroxyle terminal est substitué par un ester et/ou un éther d'alkyle en C₁-C₁₂, ladite substance étant substituée à chaque fois par au moins deux chaînes de polyéthylèneglycol de ce type.

2. Substance réagissant vis-à-vis d'une tumeur ou substance pour diagnostiquer une tumeur selon la revendication 1, caractérisée en ce que les chaînes de polyéthylèneglycol substituées en Ω ont une longueur de chaîne de n = 10 - 200, en particulier de 100 à 110.

3. Substance réagissant vis-à-vis d'une tumeur ou substance pour diagnostiquer une tumeur selon la revendication 1 ou 2, caractérisée en ce que la substance de base substituée par des chaînes de polyéthylèneglycol est un composé cis-platine, un stéroïde, une porphine, une phtalocyanine, une napthalocyanine ou un produit usuel de contraste.

4. Procédé de préparation de la substance réagissant vis-à-vis d'une tumeur ou substance pour diagnostiquer une tumeur selon les revendications 1 à 3, caractérisé en ce qu'on fait réagir la substance de base d une manière connue en soi avec un polyéthylèneglycol dont le groupe hydroxyle terminal est substitué par un groupe ester ou éther d'alkyle en C₁-C₁₂, dans un solvant approprié, et on isole, d'une manière connue en soi, la substance dérivée de cette manière.

5. Procédé selon la revendication 4, caractérisé en ce que, pour le cas où on utilise des chaînes de polyéthylèneglycol ayant un poids moléculaire supérieur à 5 000, en particulier supérieur à 10 000, on active la réaction avec la substance réagissant vis-à-vis d'une tumeur ou substance pour diagnostiquer une tumeur, au moyen de chlorure de cyanuryle, d'une manière connue en soi.

6. Utilisation d'une substance de faible poids moléculaire, réagissant vis-à-vis d'une tumeur ou servant à diagnostiquer une tumeur, à substitution polyéthylèneglycol substitué en oméga par un ester ou un éther d'alkyle en C₁-C₁₂ (degré de polymérisation = 5 - 250), contenant :
- au moins deux groupes hydroxyles phénoliques et/ou au moins deux groupes amino phénoliques, ou
- au moins un groupe hydroxyle phénolique et/ou au moins un groupe amino phénolique et au moins un groupe amino aliphatique,
pour la préparation d'un agent, de préférence concentré dans/sur un tissu tumoral, pour une utilisation en médecine nucléaire, en chimiothérapie, en diagnostic par fluorescence et en thérapie photodynamique, ainsi que dans des analyses de RMN et de CT, la substitution apparaissant sur le groupe hydroxyle phénolique et/ou sur le groupe amino phénolique et/ou sur le groupe amino aliphatique, sauf dans le cas de O-iode.

7. Utilisation de porphines, de phtalocyanines et de naphtalocyanines à substitution polyéthylèneglycol, qui sont éventuellement marquées radioactivement, selon la revendication 6.

8. Utilisation de diphosphonates à substitution polyéthylèneglycol et marqués radioactivement avec de l'iode, selon la revendication 6, en particulier pour la thérapie à l'iode radioactif avec I¹³¹ et I¹²⁵, de préférence d'abord avec I¹³¹ et ensuite avec I¹²⁵.

9. Utilisation de dérivés de cis-platine et de stéroïdes à substitution polyéthylèneglycol selon la revendication 6.

10. Utilisation de diphosphonates à substitution polyéthylèneglycol et marqués avec de l'iode radioactif selon la revendication 6.

11. Utilisation de dérivés de cis-platine et de stéroïdes à substitution polyéthylèneglycol selon la revendication 6.

12. Utilisation de peptides, de porphines, de phtalocyanines et de naphtalocyanines à substitution polyéthylèneglycol et activés avec de l'iode radioactif, du brome radioactif ou avec des tons métalliques radioactifs, selon la revendication 6.

13. Utilisation de composés fortement iodés a substitution polyéthylèneglycol, en particulier des porphines et des tyrosines polygomères selon la revendication 6.

14. Utilisation de complexes de métal à substitution polyéthylèneglycol, en particulier le gadolinium, les phtalocyanines et les naphtalocyanines, selon la revendication 6.

15. Utilisation de complexes de métal à substitution polyéthylèneglycol, en particulier les gadolinium-phtalocyanines, selon la revendication 6.

16. Utilisation de radiosensibilisateurs à substitution polyéthylèneglycol, en particulier le nitro-imidazole, selon la revendication 6.

17. Utilisation de luminols à substitution polyéthylèneglycol selon la revendication 6.

18. Utilisation d'esters de l'acide borique à substitution polyéthylèneglycol selon la revendication 6.

19. Utilisation de composés marqués avec Dy¹⁶⁴, en particulier les phtalocyanines et les naphtalocyanines, selon la revendication 6.
